# EUROPEAN PATENT SPECIFICATION

(11) **EP 0 659 096 B1**
(45) Date of publication and mention of the grant of the patent: **10.11.1999**
(21) Application number: 92920535.9
(22) Date of filing: 26.08.1992
(51) Int. Cl.: A61M 16/10

(54) **NEBULIZER APPARATUS FOR HIGH-HUMIDITY OXYGEN THERAPY**
VERNEBLUNGSVORRICHTUNG FÜR SAUERSTOFF-THERAPIE MIT HOHER FEUCHTIGKEIT
NEBULISEUR POUR L'OXYGENOTHERAPIE HUMIDE

(43) Date of publication of application: 28.06.1995
(73) Proprietor: FLOW-METER S.p.A., 24040 Levate (IT); W.L. GORE & ASSOCIATES, INC., Elkton, MD 21922 (US)
(72) Inventor: PARATICO, Roberto, I-24040 Levate (IT); GOLDBERG, Wendy W.L. Gore & Associates, Inc., Elkton, MD 21922 (US)
(74) Representative: Botti, Mario
(86) International application number: IT9200108
(87) International publication number: WO9404211

(56) References cited:
- US-A- 3 782 083
- US-A- 3 929 128
- US-A- 4 128 407

## Description

### Field of the Invention

This invention relates to a nebulizer apparatus according to the preamble of claim 1.

### Background Art

It is a known fact that, in the specific field of application of this invention, there exists a need for supplying patients or invalids subjected to such therapy with an oxygen/air mixture enriched with a water mist.

The finely divided water ensures, in fact, proper humidity of the mixture being inhaled by the patient.

It is also very important that the mixture be supplied under aseptic conditions, to prevent formation of bacterial growths that could be delivered to the patient, already in a weakened state of health, and generate new pathologies.

To meet this demand, the prior art has proposed the use of nebulizers connected to sterile water containers from which they draw through a Venturi pipe arrangement.

Such prior apparatus, while being widely employed, are not devoid of disadvantages, and fall short of fully meeting the above-outlined requirements.

In the first place, the air drawn in directly from the environment, through the Venturi arrangement, may itself be a source of bacterial load and, since bacterial and viral elements are used to grow at a fast rate in a medium having a high moisture content, it appears that prior art nebulizers can hardly be regarded as really aseptic.

Further, the use of containers or bottles of sterile water is definitely cost-intensive. In fact, this class of products for medical applications have such specific features that make them significantly expensive and certainly not universally available items.

A known solution is disclosed in the U.S. patent No. 4,128,407 which reletes to a nebulizer apparatus according to the preamble of the enclosed claim 1.

Such an apparatus includes a several layer filter pad protecting against bacteria the flow path downstream from the nebulizer.

A second solution is disclosed in the U.S. patent No. 3,929,128 relating to a nebulizer apparatus using a PTFE filter plug.

This filter apparatus prevents liquid, water and bacteria from passing. However, this solution requires anyway the use of sterile water.

A futher document, the U.S. patent No. 3,782,083, relates to a synthetic plastic material filter which however doesn't allow the passage of water vapor.

The underlying technical problem of this invention is to provide a nebulizer apparatus for oxygen therapy which such features as to obviate the above-mentioned drawbacks with which the prior art is beset.

### Disclosure of the Invention

The solutive idea of this invention is to provide an appropriate filtering by means of the features defined in claim 1.

Based on this solutive idea, the technical problem is solved by a nebulizer apparatus, as previously indicated, comprising an anti-bacterial filter, as defined in the characterising portion of claim 1, installed in the flow path of the humidified mixture downstream from the nebulizer.

Such inventive apparatus allows the use of non-sterile water in the nebulizer. This does away both with the need to provide expensive pre-filled containers of sterile water or a comprehensive anti-bacterial filter for all the mixture components.

This advantageous feature reflects in an ability to produce a sterile mixture on line with individual application requirements.

The features and advantages of a nebulizer apparatus according to the invention will become apparent from the following detailed description of an embodiment thereof, given by way of non-limitative example with reference to the accompanying drawings.

### Brief Description of Drawings

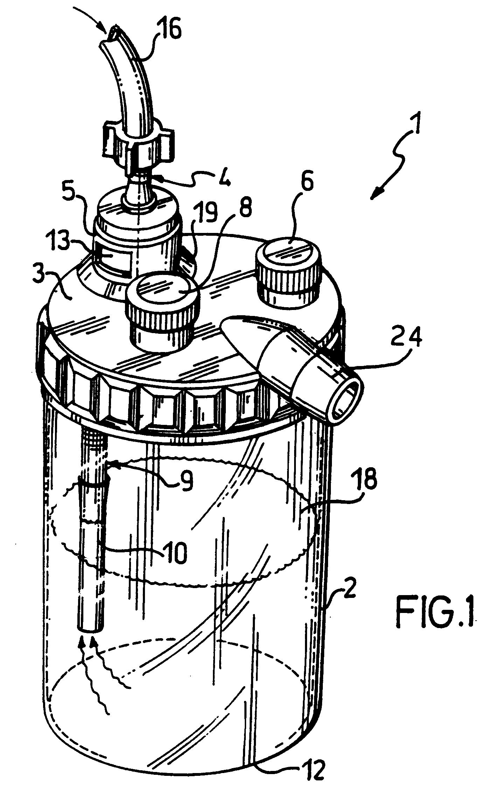

Figure 1 is a perspective view of a nebulizer apparatus according to a preferred embodiment of the invention;

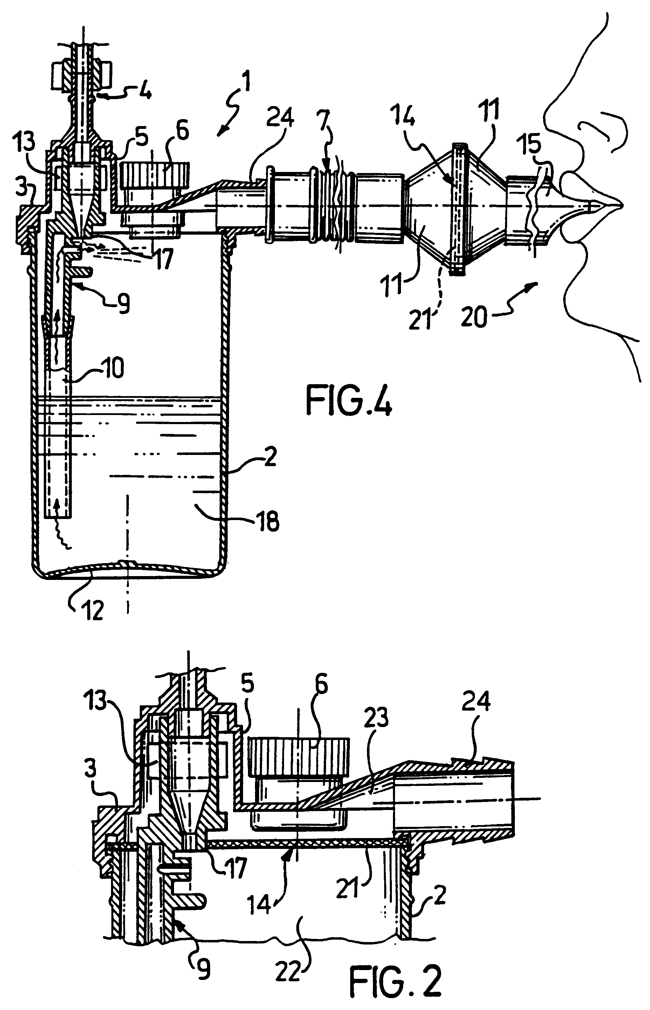

Figure 2 is a vertical and partial section view of the nebulizer in Figure 1; and
Figure 3 is a perspective view of a further embodiment of a nebulizer apparatus according to the invention;
Figure 4 shows schematically and in vertical section a detail of the figure 3 embodiment, shown in operation.

### Modes for Carrying Out the Invention

With reference to the drawing figures, generally shown at 1 is a nebulizer apparatus for oxygen therapy according to the invention and particularly, though not exclusively, intended for medical applications in a high-humidity sterile medium.

The nebulizer 1 comprises a rigid container 2, basically cup-shaped, which is formed preferably from polycarbonate and has a closure cover 3 threaded removably thereon.

The cup 2 contains water 18 for nebulization useful to prepare a high-humidity gaseous oxygen/air mixture to be administered to a patient 20 undergoing intensive therapy or care.

Provided on the cover 3 is a first fitting 4 for connection to an oxygen supply, not shown because conventional. The connection of the supply to the fitting would be established by means of a tube 16.

The fitting 4 is incorporated to a Venturi device 5 whereby ambient air is admixed to the oxygen from said supply. The incoming air flows through a port 13 which cap be restricted by means of a ring nut 19.

Beneath the cover 3, the Venturi device 5 is in fluid communication with a slightly offset nebulizer 9 having an extension tube 10 fitted thereover for admitting water to be nebulized.

This tube 10 draws from a point close to the bottom 12 of the cup 2.

Provided on the nebulizer 9 is also a choke device 17 effective to boost the velocity of the mixture flow to the patient 20.

The cover 3 includes additional fittings, and specifically at least one water filler cap 6 and a second inlet 8 for optional insertion of a resistance heater.

Advantageously, the apparatus 1 further comprises a delivery conduit 7 which has one end connected to the cover 3, through a connection element 24, and is in fluid communication with the interior of the cup 2. The other end 15 of the conduit 7 is free and adapted to be presented to the patient 20.

Conveniently included in the fluid path addressed to the patient, an anti-bacterial sterilizing filter 14 is provided according to the invention.

The filter 14 includes a hydrophobic or water-repellent membrane 21 formed from a very thin sheet of a microporous plastics material.

More specifically, the membrane 21 is mounted inside the container 2 and associated with the cover 3.

The membrane 21 is secured around the inner peripheral edge of the cover 3 in order to establish an aseptic barrier between the nebulizer 9 and the port in the cover 3 for the delivery conduit 7.

Thus, a nebulization chamber 22 is defined within the container 2 which extends between the free surface of the water 18 and the membrane 21. Defined within the cover 3 is a receiving header 23 for the humidified mixture. This header 23 directs the mixture to the port in the cover 3 for the conduit 7.

The membrane 21 is formed from polytetrafluoroethylene (PTFE) and has preferably a pore size of less than 10 µm (0.01 mm).

A peculiar feature of this membrane 21 is that it allows a large flow of humid air through its pores, but blocks particles, particulate matter, bacteria, and aerosol droplets.

In essence, the membrane 21 allows only humid air/oxygen to pass through and prevent aerosol droplets from reaching the patient.

The membrane 21 also arrests bacteria, viruses, and any corpuscles other than in a gaseous state.

Actual tests have shown that the membrane 21 can function as a filter with an efficiency in excess of 99.9999% for removal of particles 0.1 µm and larger.

The membrane is preferably realized with a very fine fibril structure known with the trademark "GORE-TEX".

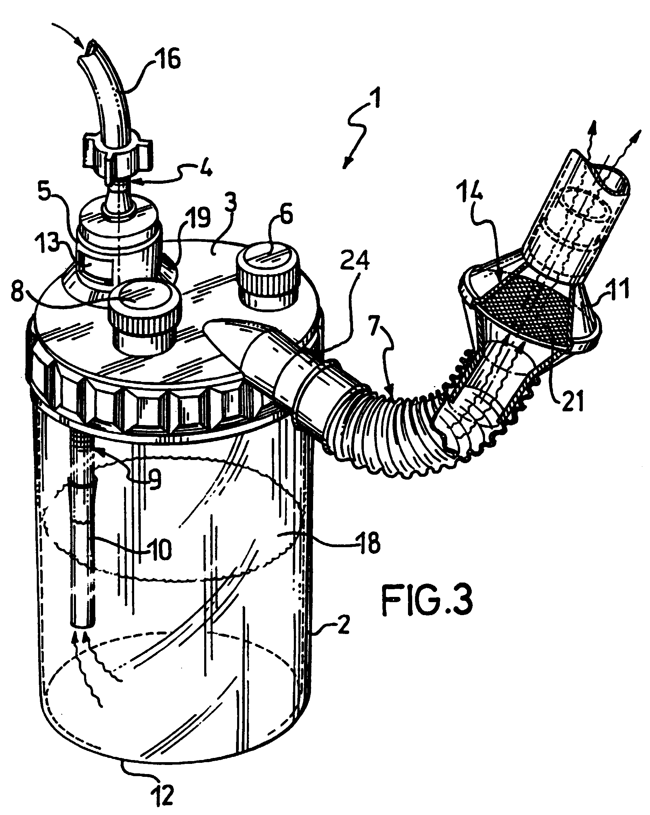

With specific reference to the embodiment shown in Figure 3, a further embodiment of the nebulizer apparatus according to the invention will now be described wherein parts and co-operating portions previously illustrated carry the same reference numerals.

In this variant, the anti-bacterial sterilizing filter 14 is conveniently included in the fluid path bounded by the conduit 7 and housed within a sealed case.

More particularly, the filter 14 is housed within a case formed of a pair of funnel-shaped members 11 being identical with each other and sealed around the peripheral edge of their flared portion, as shown in figure 4.

This case is connected in the conduit 7 by a quick-connect arrangement including oppositely directed hose attachment ends.

The membrane 21 is held centrally between the opposed shells represented by the two sealing members 11.

A primary advantage of the inventive apparatus is that it allows for the use of non-sterile water in the nebulizer.

This does away both with the need to provide expensive pre-filled containers of sterile water or comprehensive anti-bacterial filter for all the mixture components.

This advantageous feature reflects in an ability to produce a sterile mixture on line with individual application requirements.

In other words, the apparatus of this invention can be "tailored" to the individual patient.

Another major advantage is that the membrane would in any case he associated with the apparatus cover 3, thereby the water chamber 18 in the cup 2 can be filled without interfering with the aseptic barrier 21.

In addition, the filter membrane is inserted removably in the mixture delivery conduit to the patient, and accordingly, easily replaced for customary maintenance and cleaning operations.

Understandably, the apparatus of this invention may be modified and altered within the scope of the following claims.

## Claims

1. A nebulizer apparatus (1) for high-humidity oxygen therapy comprising a sealed container (2) for containing water (18) for nebulization, a nebulizer (9) drawing from the container (2) and being in fluid communication with a supply of a gaseous mixture, and an anti-bacterial filter (14) installed in a delivery conduit (7) in the flow path of said humidified mixture downstream from the nebulizer (9), characterized in that said anti-bacterial filter (14) comprises a hydrophobic, water repellant and bacteria repellant membrane (21) of microporous PTFE formed by a very fine fibrils structure which allows the passage of an effective flow of humid air while preventing the passage of aerosol particles in said delivery conduit thereby allowing the use of non-sterile water in the nebulizer.

2. A nebulizer apparatus according to Claim 1, characterized in that said membrane (21) is formed from a thin sheet of a synthetic microporous PTFE plastics material having a smaller pore size than 0.01 millimetres.

3. A nebulizer apparatus according to Claim 1, characterized in that said filter (14) is housed within a case (11) which fits removably in the delivery conduit (7) by means of a quick-connect arrangement.

4. A nebulizer apparatus according to Claim 1, characterized in that said container is closed by a cover and the anti-bacterial filter (14) is attached to said cover (3) providing a barrier between the nebulizer (9) and the delivery conduit (7).

## Patentansprüche

1. Zerstäubergerät (1) für die Sauerstofftherapie mit hoher Feuchtigkeit, umfassend einen abgedichteten Behälter (2) zur Aufnahme von zu zerstäubendem Wasser (18), einen Zerstäuber (9), der aus dem Behälter (2) abzieht und in Fluidverbindung mit einer Quelle eines gasförmigen Gemisches steht, und ein antibaterielles Filter (14), welches in einer Förderleitung (7) im Strömungsweg des angefeuchteten Gemisches stromabwärts bezüglich des Zerstäubers (9) angebracht ist, dadurch gekennzeichnet, daß das antibaterielle Filter (14) eine hydrophobe, wasserabweisende und bakterienabweisend Membran (21) aus mikroporösem PTFE aufweist, gebildet durch eine Struktur feiner Fibrillen, die den Durchgang eines effektiven Stroms feuchter Luft gestattet und gleichzeitig den Durchgang von Aerosolpartikeln in der Förderleitung verhindert, um dadurch die Verwendung nicht-sterilen Wassers in dem Zerstäuber zu gestatten.

2. Zerstäubergerät nach Anspruch 1, dadurch gekennzeichnet, daß die Membran (21) aus einem dünnen Flachstück eines synthetischen mikroporösen PTFE-Kunststoffmaterial mit einer Porengröße von weniger als 0,01 mm gebildet wird.

3. Zerstäubergerät nach Anspruch 2, dadurch gekennzeichnet, daß das Filter (14) in einem Gehäuse (11) untergebracht ist, welches herausnehmbar in die Förderleitung (7) mittels einer Schnellverbinderanordnung einsetzbar ist.

4. Zerstäubergerät nach Anspruch 1, dadurch gekennzeichnet, daß der Behälter von einem Deckel verschlossen wird und das antibakterielle Filter (14) in dem Deckel (3) befestigt ist, um eine Sperre zwischen dem Zerstäuber (9) und der Förderleitung (7) zu bilden.

## Revendications

1. Appareil (1) formant nébuliseur pour une oxygénothérapie à haute humidité comprenant un récipient hermétique (2) pour contenir de l'eau (18) pour nébulisation, un nébuliseur (9) fonctionnant par aspiration à partir du récipient (2) et étant en communication fluidique avec une alimentation d'un mélange gazeux, et un filtre antibactérien (14) monté dans un conduit d'alimentation (7) dans la trajectoire de flux dudit mélange humidifié en aval du nébuliseur (9),
caractérisé en ce que ledit filtre antibactérien (14) comprend une membrane (21) hydrophobe, hydrofuge et antibactérienne en polytétrafluoroéthylène (PTFE) microporeux formé par une structure fibrillaire très fine qui permet le passage d'un flux efficace d'air humide, tout en empêchant le passage de particules d'aérosol dans ledit conduit d'alimentation, permettant ainsi l'utilisation d'eau non stérile dans le nébuliseur.

2. Appareil formant nébuliseur selon la revendication 1, caractérisé en ce que ladite membrane (21) est formée d'une feuille mince d'une matière plastique synthétique à base de polytétrafluoroéthylène (PTFE) poreux ayant une dimension de pores inférieure à 0,01 millimètre.

3. Appareil formant nébuliseur selon la revendication 1, caractérisé en ce que ledit filtre (14) est logé à l'intérieur d'un boîtier (11) qui est monté de façon amovible dans le conduit d'alimentation (7) au moyen d'un dispositif à raccord rapide.

4. Appareil formant nébuliseur selon la revendication 1, caractérisé en ce que ledit récipient est fermé par un couvercle et en ce que le filtre antibactérien (14) est fixé sur ledit couvercle (3) en formant une barrière entre le nébuliseur (9) et le conduit d'alimentation (7).
